Europäisches Patentamt

European Patent Office

Office européen des brevets

(19)

(11) Veröffentlichungsnummer: **0 133 258**
**B1**

(12) # EUROPÄISCHE PATENTSCHRIFT

(45) Veröffentlichungstag der Patentschrift:
06.03.89

(21) Anmeldenummer: 84108606.9

(22) Anmeldetag: 20.07.84

(51) Int. Cl.⁴: **A 61 K 31/355**, A 61 K 47/00

(54) Injektionslösungen für Vitamin E.

(30) Priorität: 22.07.83 CH 4029/83
06.06.84 CH 2747/84

(43) Veröffentlichungstag der Anmeldung:
20.02.85 Patentblatt 85/8

(45) Bekanntmachung des Hinweises auf die Patenterteilung:
06.03.89 Patentblatt 89/10

(84) Benannte Vertragsstaaten:
AT BE CH DE FR GB IT LI NL

(56) Entgegenhaltungen:
FR-A-2 358 161
US-A-3 197 368
US-A-4 115 313

Die Akte enthält technische Angaben, die nach dem Eingang der Anmeldung eingereicht wurden und die nicht in dieser Patentschrift enthalten sind.

(73) Patentinhaber: F. HOFFMANN- LA ROCHE & CO.
Aktiengesellschaft, CH- 4002 Basel (CH)

(72) Erfinder: Ferro, Alberto, Dr., Gstaltenrainweg 67,
CH- 4125 Riehen (CH)
Erfinder: Steffen, Hans, Dr., Olsbergerstrasse 27,
CH- 4411 Arisdorf (CH)

(74) Vertreter: Lederer, Franz, Dr., Van der Werth,
Lederer & Riederer Patentanwälte Lucile- Grahn-
Strasse 22, D-8000 München 80 (DE)

EP 0 133 258 B1

## Beschreibung

Die vorliegende Erfindung betrifft wässrige Mischmicell-Lösungen von Vitamin E, Phospholipiden, und Gallensäuren-Salzen.

Es ist bekannt, dass Vitamin E in Mischmicellen von Gallensäuren und Lecithinen gelöst werden kann. Diese bekannten Lösungen enthalten nur geringe Mengen Vitamin E. In der US-Patentschrift 3 197 368 werden Micellösungen von Lecithinen und Gallensäuren beschrieben, die bis zu 2 % Vitamin E ($\alpha$-Tocopherol) bezogen auf die Lecithinmenge enthalten können. In der deutschen Auslegeschrift 2 433 173 werden Micellösungen von Phospholipiden und Glycocholsäure beschrieben, die 10 mg Vitamin E und 5 g Phospholipid pro Liter enthalten. In der FR-Patentschrift 2 358 161 wird eine Vitamin $K_1$-Lösung in Phospholipid-Gallensäure-Mischmicellen beschrieben, die 112 g Vitamin $K_1$ pro Mol Lecithin enthält.

Es bestand, z. B. zur Herstellung von Injektionslösungen, ein Bedürfnis, über wesentlich konzentriertere Lösungen von Vitamin E zu verfügen.

Die erfindungsgemässen wässrigen Vitamin E-Mischmicell-Lösungen sind dadurch gekennzeichnet, dass das Molverhältnis Phospholipid : Gallensäure 0,2-0,9 : 1 ist und die Lösungen mindestens etwa 100 g Vitamin E pro Mol Phospholipid enthalten, vorzugsweise enthalten die Lösungen 10 - 50 g Vitamin E pro Liter.

Vorzugsweise ist das Molverhältnis Phospholipid Gallensäure 0,25-0,5 : 1.

Als Gallensäuren kommen für die erfindungsgemässen Lösungen Trihydroxycholansäuren, wie Cholsäure, Glycocholsäure, Taurocholsäure, und Dihydroxycholansäuren, wie Deoxycholsäure, Glycodeoxycholsäure, Taurodeoxycholsäure, Chenodeoxycholsäure, Glycochenodeoxycholsäure und Taurochenodeoxycholsäure, in Betracht. Bevorzugt ist Glycocholsäure. Als Gallensäuresalze kommen insbesondere Alkalisalze, wie das Natriumsalz, in Betracht.

Beispiele von Phospholipiden sind Phosphatide, wie Phosphatidylcholine, Glycerinäther-Phosphatide, Phosphatidyläthanolamin, Phosphatidylinosit, Phosphatidylserin, Plasmologene oder Sphingomyeline. Bevorzugt sind Phosphatidylcholine, wie Soja- und Eilecithin. Der Ausdruck "Vitamin E" soll optisch aktives und racemisches Vitamin E ($\alpha$-Tocopherol) umfassen.

Die erfindungsgemässen Lösungen können isotonisierende Zusätze enthalten, um sie für Injektionszwecke geeignet zu machen.

Als isotonisierende Zusätze kommen insbesondere in Betracht: Physiologische Kochsalz- und Glucoselösung, Tris-Puffer, Phosphat-Puffer, Citrat-Puffer, Glycin-Puffer, Citrat-Phosphat-Mischpuffer, usw. Der osmotische Druck der erfindungsgemässen Injektionslösungen sollte annähernd demjenigen des Blutes entsprechen, d.h. etwa 300 mOsm betragen, kann aber in gewissen Grenzen variieren.

Die erfindungsgemässen Lösungen können weitere Zusätze, z. B. Konservierungsmittel und Stabilisatoren, sowie andere pharmazeutische Wirkstoffe, z. B. andere Vitamine wie Vitamin A, D, Biotin, Vitamine der B-Gruppe und Vitamin C enthalten. Zur Verbesserung der Stabilität können die erfindungsgemässen Lösungen z. B. Äthanol enthalten.

Die erfindungsgemässen Lösungen können in für die Herstellung von Mischmicell-Lösungen an sich bekannter Weise hergestellt werden, beispielsweise dadurch, dass das Phospholipid, das Gallensäure-Salz und das Vitamin in einem organischen Lösungsmittel gelöst werden, darauf das organische Lösungsmittel eingedampft und hierauf das Wasser und gegebenenfalls isotonisierende Zusätze und weitere Ingredienzien zugegeben werden. Als organische Lösungsmittel kommen solche in Betracht, in denen die zu lösenden Komponenten hinreichend löslich sind, wie z. B. niedere Alkanole, insbesondere Äthanol.

Ein besonders bevorzugtes Verfahren besteht darin, dass man eine Gallensäure, vorzugsweise Glycocholsäure, in einer äthanolischen Lösung von Vitamin E und Lecithin suspendiert und danach eine zur Überführung der Gallensäure in ein Salz erforderliche Menge einer Base, z. B. Natronlauge, zusetzt. Zweckmässig beträgt die Äthanolmenge 6 - 8 Gewichts-% bezogen auf das Gesamtvolumen der Lösung.

Die so erhaltenen Lösungen können sterilisiert und/oder lyophilisiert werden.

Die Erfindung wird durch die nachfolgenden Beispiele weiter erläutert:

## Beispiel 1

1,8 ml einer 10 gew.-%-igen methanolischen Lösung von d,1-$\alpha$-Tocopherol, 0,76 ml einer 25 gew.-%-igen äthanolischen Lösung von PHOSPHOLIPON 100 (Sojalecithin) und 3,9 ml einer 10 gew.-%-igen methanolischen Natriumglykocholatlösung werden miteinander vermischt. Die Lösungsmittel werden bei 40°C entfernt und der Rückstand wird mit 3,24 ml Wasser versetzt und gerührt, wobei eine klare Lösung erhalten wird. Die Lösung wird steril filtriert, im Ampullen abgefüllt und bei 120°C 20 Minuten sterilisiert.

## Beispiel 2

50,0 mg d,1-$\alpha$-Tocopherol werden in 70,0 mg Äthanol gelöst. Danach werden 53,4 mg Lecithin in der so erhaltenen Lösung gelöst. In dieser Lösung werden 88,5 mg Glycocholsäure sorgfältig suspendiert. Danach werden 506,5 µl 1,5 %-ige (Gew./Vol.) Natronlauge zugegeben. Man rührt, bis eine klare Lösung erhalten wird und stellt dann durch Zusatz von 1N Salzsäure auf pH 6 und füllt mit Wasser ad. inj. auf 1,0 ml auf.

## Patentansprüche

1. Wässrige Mischmicell-Lösungen von Vitamin E, Phospholipiden, und Gallensäuren-Salzen, dadurch gekennzeichnet, dass das Molverhältnis Phospholipid: Gallensäure 0,2-0,9 : 1 ist und die Lösungen mindestens 100 g Vitamin E pro Mol Phospholipid enthalten; und Lyophilisate davon.

2. Lösungen gemäss Anspruch 1, dadurch gekennzeichnet, dass das Molverhältnis Phospholipid: Gallensäure 0,25-0,5 : 1 ist.

3. Lösungen gemäss den Ansprüchen 1 oder 2, dadurch gekennzeichnet, dass sie 10 - 50 g Vitamin E pro Liter enthalten.

4. Lösungen gemäss den Ansprüchen 1-3, dadurch gekennzeichnet, dass sie 6 - 8 Gewichts-% Äthanol bezogen auf das Gesamtvolumen enthalten.

5. Verfahren zur Herstellung der Lösungen gemäss den Ansprüchen 1 - 3, dadurch gekennzeichnet, dass man das Phospholipid, das Gallensäure-Salz und das Vitamin in einem organischen Lösungsmittel löst, das Lösungsmittel eindampft und hierauf Wasser zusetzt.

6. Verfahren nach Anspruch 5, dadurch gekennzeichnet, dass das Molverhältnis Phospholipid: Gallensäure 0,25-0,5 : 1 ist.

7. Verfahren nach Anspruch 5 oder 6, dadurch gekennzeichnet, dass die Lösungen 10 - 50 g Vitamin E pro Liter enthalten.

8. Verfahren nach einem der Ansprüche 5 - 7, dadurch gekennzeichnet, dass man eine Gallensäure in einer äthanolischen Lösung von Vitamin E und Lecithin suspendiert und danach eine zur Überführung der Gallensäure in ein Salz erforderliche Menge einer Base zusetzt, wobei die Äthanolmenge 6 - 8 Gewichts-% bezogen auf das Gesamtvolumen der Lösung beträgt.

## Claims

1. Aqueous mixed micelle solutions of vitamin E, phospholipids and cholanic acid salts, characterized in that the molar ratio phospholipid cholanic acid is 0.2-0.9 : 1 and the solutions contain at least 100 g of vitamin E per mol of phospholipid; and lyophilizates thereof.

2. Solutions in accordance with claim 1, characterized in that the molar ratio phospholipid: cholanic acid is 0.25-0.5 : 1.

3. Solutions in accordance with claims 1 or 2, characterized in that they contain 10 - 50 g of vitamin E per litre.

4. Solutions in accordance with claims 1 - 3, characterized in that they contain 6 - 8 weight % of ethanol based on the total volume.

5. A process for the manufacture of the solutions in accordance with claims 1 - 3, characterized by dissolving the phospholipid, the cholanic acid salt and the vitamin in an organic solvent, evaporating the solvent and thereupon adding water.

6. A process according to claim 5, characterized in that the molar ratio phospholipid cholanic acid is 0.25-0.5 : 1.

7. A process according to claim 5 or 6, characterized in that the solutions contain 10 - 50 g of vitamin E per litre.

8. A process according to any one of claims 5 - 7, characterized in that the cholanic acid is suspended in an ethanolic solution of vitamin E and lecithin and thereafter an amount of base which is required to convert the cholanic acid into a salt is added, whereby the amount of ethanol is 6 - 8 weight % based on the total volume of the solution.

## Revendications

1. Solutions aqueuses micellaires, à micelles mélangées, de vitamine E, de phospholipides et de sels d'acides biliaires, caractérisées en ce que le rapport molaire phospholipide/acide biliaire est de 0,2 à 0,9 : 1 et en ce que les solutions contiennent au moins 100 g de vitamine E par mole de phospholipide; et leurs lyophilisats.

2. Solutions selon la revendication 1, caractérisées en ce que le rapport molaire phospholipide/acide biliaire est de 0,25 à 0,5 : 1.

3. Solutions selon les revendications 1 ou 2, caractérisées en ce qu'elles contiennent de 10 à 50 g de vitamine E par litre.

4. Solutions selon les revendications 1 à 3, caractérisées en ce qu'elles contiennent de 6 à 8 % en poids d'éthanol, par rapport au volume total.

5. Procédé de préparation des solutions selon les revendications 1 à 3, caractérisé en ce que l'on dissout le phospholipide, le sel d'acide biliaire et la vitamine dans un solvant organique, on évapore le solvant puis on ajoute de l'eau.

6. Procédé selon la revendication 5, caractérisé en ce que le rapport molaire phospholipide/acide biliaire est de 0,25 à 0,5 : 1.

7. Procédé selon la revendication 5 ou 6, caractérisé en ce que les solutions contiennent de 10 à 50 g de vitamine E par litre.

8. Procédé selon l'une des revendications 5 à 7, caractérisé en ce que l'on met en suspension un acide biliaire dans une solution éthanolique de vitamine E et de lécithine puis on ajoute une base à la quantité nécessaire pour convertir l'acide biliaire en un cel, la quantité d'éthanol étant de 6 à 8 % en poids, par rapport au volume total de la solution.